# EUROPEAN PATENT APPLICATION

(11) **EP 4 431 087 A1**
(43) Date of publication of application: **18.09.2024**
(21) Application number: 24163558.0
(22) Date of filing: 14.03.2024
(51) Int. Cl.: A61K 9/20, A61K 9/28

(54) **A FILM COATED TABLET OF SITAGLIPTIN OR A PHARMACEUTICALLY ACCEPTABLE SALT THEREOF**

(30) Priority: 14.03.2023 TR 202302813
(71) Applicant: SANOVEL ILAC SANAYI VE TICARET A.S., Istanbul 34460 (TR)
(72) Inventor: SUNEL, Fatih, Istanbul (TR); BILGEHAN ATAK, Fadime, Istanbul (TR); ATAMAN, Seval, Istanbul (TR); YAZICI, Ozlem, Istanbul (TR); SARP, Onder, Istanbul (TR)
(74) Representative: Sevinç, Erkan

(57) **Abstract**

The present invention relates to a film coated tablet comprising sitagliptin or a pharmaceutically acceptable salt thereof and at least two fillers and at least two lubricants wherein, lubricants are magnesium stearate and sodium stearyl fumarate. The present invention also relates to a simple, rapid, cost effective, time-saving and industrially convenient method of preparing the formulation.

## Description

### Field of the Invention

The present invention relates to a film coated tablet comprising sitagliptin or a pharmaceutically acceptable salt thereof and at least two fillers and at least two lubricants wherein, lubricants are magnesium stearate and sodium stearyl fumarate. The present invention also relates to a simple, rapid, cost effective, time-saving and industrially convenient method of preparing the formulation.

### Background of the Invention

Diabetes mellitus is a group of disorders of carbohydrate metabolism in which the action of insulin is diminished or absent through altered secretion, decreased insulin activity or a combination of both factors. There are two main types of diabetes; Type 1 and Type 2:
Type 1 diabetes occurs because the insulin-producing cells of the pancreas (beta cells) are damaged. In Type 1 diabetes, the pancreas makes little or no insulin, so sugar cannot get into the body's cells for use as energy. People with Type 1 diabetes must use insulin injections to control their blood glucose.

In Type 2 diabetes, the pancreas makes insulin, but it either doesn't produce enough, or the insulin does not work properly. This diabetes occurs most often in people who are over 40 years old and overweight. Type 2 diabetes may sometimes be controlled with a combination of diet, weight management, and exercise. However, treatment also may include oral glucose-lowering medications or insulin injections.

Sitagliptin is indicated as an adjunct to diet and exercise to improve glycemic control in adults with type 2 diabetes mellitus. It is an oral antihyperglycemic of the dipeptidyl peptidase-4 (DPP-4) inhibitor class. DPP-4 inhibitors work by blocking the action of DPP-4, an enzyme which destroys the hormone incretin. There are two types of incretin hormones found in the body, called glucagon-like peptide-1 (GLP-1) and glucose-dependent insulinotropic peptide (GIP). These hormones are naturally produced by the body in response to food intake. Their function is to help the body produce more insulin only when it is needed and reduce the amount of glucose being produced by the liver when it is not needed. Sitagliptin works by binding to DPP-4 and preventing it from breaking down the GLP-1 and GIP. This increases the levels of these hormones in the body and so increases their effect on controlling blood sugar.

The chemical name of sitagliptin is (R) -4-oxo-4- [3- (trifluoromethyl) -5,6-dihydro [1,2,4] triazolo [4,3-a] pyrazin-7 (8H)-yl]-1- (2,4,5-trifluorophenyl) butan-2-amine), and the chemical structure of sitagliptin is shown in Formula 1.

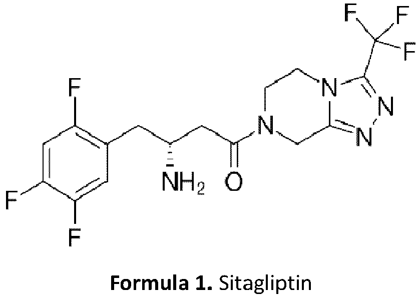

Sitagliptin is disclosed in the patent US6699871. A crystal phosphate monohydrate form of sitagliptin is disclosed in the patent WO2005003135.

Sitagliptin increases plasma GLP-1 concentration and elevates cellular cAMP levels in pancreatic beta-cells leading to potentiate insulin secretion, whereas metformin improves glucose tolerance in patients with Type 2 diabetes, lowering both basal and postprandial plasma glucose. Its pharmacologic mechanisms of action is different from other classes of oral antihyperglycemic agents in that metformin decreases hepatic glucose production, decreases intestinal absorption of glucose and improves insulin sensitivity by increasing peripheral glucose uptake and utilization. Therefore, they both help to reduce blood glucose levels in different pathways in type 2 diabetes patients.

TR2016/10368 relates to a pharmaceutical film tablet formulation comprising 31-32% w/w of sitagliptin phosphate anhydrous wherein the pharmaceutically excipients are microcrystalline cellulose, calcium hydrogen phosphate anhydrous, croscarmellose sodium, sodium stearyl fumarate and magnesium stearate wherein the tablet is obtained by direct compression method. Moreover, 14 to 16% w/w microcrystalline cellulose is used as diluent agent.

CN114159401 (A) discloses a sitagliptin phosphate tablet and a preparation method thereof, the tablet comprises 20-50% of sitagliptin phosphate, 50-80% of a diluent, 3-10% of a disintegrating agent and 1-10% of a lubricant. The tablet is obtained by wet granulation.

Given that the greatest problem encountered with a film coated tablet of sitagliptin or a pharmaceutically acceptable salt thereof (sitagliptin phosphate monohydrate or sitagliptin malate) is the lower stability of the product due to its hygroscopic feature, there is still a need in the art for a film coated tablet that provides high stability and thus a long shelf life, without compromising its dissolution, disintegration and flowability features while having a safe composition for use in hypertensive and cardiac patients.

The main object of the present invention is to obtain a film coated tablet of sitagliptin or a pharmaceutically acceptable salt thereof which overcomes all the above-mentioned problems and brings additional advantages to the relevant prior art.

### Detailed Description of the Invention

The main object of the present invention is to provide a film coated tablet comprising a therapeutically effective amount of sitagliptin or a pharmaceutically acceptable salt thereof with the desired high stability.

Another object of the present invention is to provide a film coated tablet comprising sitagliptin or a pharmaceutically acceptable salt thereof with improved flowability, compressibility, homogeneity and the desired dissolution profile.

Another object of the present invention is to provide a film coated tablet comprising sitagliptin or a pharmaceutically acceptable salt thereof prepared by simple, easy, time-saving and fast manufacturing methods.

As it is known, the use of lubricant provides excellent flowability of powders during process comprising sitagliptin. We have also seen in the present invention that the use of sodium stearyl fumarate and magnesium stearate shows good stability to the tablet.

According to one embodiment of this invention, a film coated tablet comprises;
- Sitagliptin or a pharmaceutically acceptable salt thereof,
- At least two fillers,
- At least two lubricants,

Wherein, lubricants are magnesium stearate and sodium stearyl fumarate. The invention provides the desired stability and tablet compressibility of the tablets.

According to one embodiment of the present invention, the amount of sitagliptin or a pharmaceutically acceptable salt thereof is present between 20.0% and %45.0 by weight in the total core tablet. Preferably, the amount of sitagliptin or a pharmaceutically acceptable salt thereof is between 26.0% and 38.0% by weight in the total core tablet.

According to one embodiment of the present invention, preferably, sitagliptin is in the form of phosphate monohydrate or malate.

Suitable fillers are selected from the group comprising microcrystalline cellulose, dicalcium phosphate anhydrous, lactose, lactose monohydrate, spray-dried lactose monohydrate, talc, anhydrous lactose, pregelatinized starch, mannitol, corn starch, maltodextrin, dicalcium phosphate, ammonium alginate, calcium carbonate, calcium phosphate, calcium phosphate dehydrate, neutral pellets, cellulose, magnesium oxide, maltodextrin, medium chain triglycerides, polydextrose, polymethacrylates, sodium alginate, starch, sugar sphericals, polysorbate 80 or mixtures thereof.

According to an embodiment of the present invention, the amount of fillers are 50.0% to 75.0% by weight in the total core tablet. Preferably, it is between 57.0% to 68.0% by weight in the total core tablet. The use of high amount filler has a good effect both for the dissolution profile and for powder homogeneity, and tablet compressibility.

According to one embodiment of the present invention, the fillers are microcrystalline cellulose and dicalcium phosphate anhydrous.

According to one embodiment of the present invention, the ratio of microcrystalline cellulose to dicalcium phosphate anhydrous is between 0.8:1 and 2.2:1.

Sitagliptin or a pharmaceutically acceptable salt thereof has hygroscopic feature. Therefore, the particle size of the active substance used is also important. We encountered flowability problems in micronized sitagliptin. We obtained the desired flowability and homogeneity values with the particle size d (0.9) values described below.

The term "Dx" as used herein means that x% of the particles in a composition (based on volume) have a diameter of or below a specified d value. D (0.9)" or "d90" means that the size at which %90 by volume of the particles is finer. The volume mean particle size of the compound of the Sitagliptin is determined using Malvern Mastersizer 2000 laser diffraction particle size analyzer.

According to one embodiment of the present invention, Sitagliptin or a pharmaceutically acceptable salt thereof has a d (0.9) particle size more than 75 µm. These particle size helps to provide the flowability and powder homogenization.

According to one embodiment of the present invention, Sitagliptin or a pharmaceutically acceptable salt thereof has a d (0.9) particle size more than 89 µm.

According to one embodiment of the present invention, Sitagliptin or a pharmaceutically acceptable salt thereof has a d (0.9) particle size between 88 µm and 250 µm.

According to one embodiment of the present invention, Sitagliptin or a pharmaceutically acceptable salt thereof has a d (0.9) particle size between 94 µm and 220 µm, preferably between 100 µm and 200 µm, preferably between 124 µm and 180 µm, preferably between 140 µm and 160 µm.

According to one embodiment of the present invention, Sitagliptin phosphate monohydrate has a d (0.9) particle size between 165 µm and 250 µm.

According to one embodiment of the present invention, Sitagliptin malate has a d (0.9) particle size between 75 µm and 150 µm.

According to this embodiment of the present invention, the film coated tablet comprises at least one disintegrant.

Suitable disintegrants are selected from the group comprising sodium starch glycolate, croscarmellose sodium, crospovidone, magnesium aluminum silica, sodium carboxymethyl cellulose, calcium silicate, carboxymethyl cellulose, calcium carboxymethyl cellulose, low substituted hydroxypropyl cellulose, poloxamer, sodium alginate, sodium glycine carbonate, sodium dodecyl sulfate or mixtures thereof.

According to this embodiment of the present invention, the amount of disintegrants is between 1.0% and 10.0% by weight of the total core tablet. Preferably, the amount of disintegrants is between 1.0% and 7.0% by weight of the total core tablet.

According to this embodiment of the invention, the disintegrant is croscarmellose sodium.

According to an embodiment of the present invention, the tablet comprises;
a) Sitagliptin Phosphate Monohydrate or Sitagliptin Malate
b) Microcrystalline Cellulose
c) Dicalcium Phosphate Anhydrous
d) Croscarmellose sodium
e) Magnesium stearate
f) Sodium stearyl fumarate

According to this preferred embodiment, the total composition comprises the following:
20.0-45.0% by weight of Sitagliptin Phosphate Monohydrate or Sitagliptin Malate
25.0-45.0% by weight of microcrystalline cellulose
15.0-35.0% by weight of dibasic calcium phosphate anhydrate
1.0-10.0% by weight of croscarmellose sodium
0.1-4.0% by weight of sodium stearyl fumarate
0.1-3.0% by weight of magnesium stearate by weight of the total core tablet.

However, Sitagliptin or a pharmaceutically acceptable salt thereof has hygroscopic feature. In addition to affecting the efficacy of drugs, impurities may increase the risk of toxic and side effects. In this invention, to eliminate this problem, a process which is free of solvent is preferred in terms of the desired stability. In using process, sitagliptin is not exposed to moisture and solvents. Thanks to this way eliminates the problems stability of sitagliptin and provides additional advantages to the relevant field of art.

According to one embodiment of the present invention, the film coated tablets are obtained by a process which is free of solvent.

According to one embodiment of the present invention, the film coated tablet comprising sitagliptin or a pharmaceutically acceptable salt thereof is obtained by dry granulation or direct compression process. This process helps to provide the stability of the tablets.

According to one embodiment of the present invention, a process for the preparation of the film coated tablet comprises;
a) Sieving sitagliptin or a pharmaceutically acceptable salt thereof, microcrystalline cellulose, dicalcium phosphate anhydrous and croscarmellose sodium through 630 µm and mixing,
b) Sieving magnesium stearate and sodium stearyl fumarate through 630 µm and mixing with the mixture at step (a),
c) Compressing the mixture into the tablet,
d) Coating the tablets with film coating agent.

According to this embodiment, the film coating comprises hypromellose, triacetin, fumed silica, titanium dioxide, white wax, iron oxides (red, black, yellow) and caprylic capric triglyceride.

According to one embodiment, the film coating comprises polyvinyl alcohol, talc, calcium carbonate, sodium lauryl sulphate, iron oxides (yellow, red) and ferric oxide.

According to this embodiment, the following exemplary formulations can be used in tablet compositions of the invention.

### Example 1: Film-coated tablet

| **Components** | **Amount (%)** |
|---|---|
| Sitagliptin Phosphate Monohydrate or Sitagliptin Malate | 20.0 - 45.0 |
| Microcrystalline cellulose | 25.0 - 45.0 |
| Dicalcium Phosphate Anhydrous | 15.0 - 35.0 |
| Croscarmellose sodium | 1.0 - 10.0 |
| Magnesium Stearate | 0.1 - 3.0 |
| Sodium stearyl fumarate | 0.1 - 4.0 |
| **Total core tablet** | **100** |
| Film coating | 2.5-6.0 |

### Example 2: Film-coated tablet

| **Components** | **Amount (%)** |
|---|---|
| Sitagliptin Phosphate Monohydrate | 32.12 |
| Microcrystalline cellulose | 30.93 |
| Dicalcium Phosphate Anhydrous | 30.95 |
| Croscarmellose sodium | 2.0 |
| Magnesium Stearate | 1.0 |
| Sodium stearyl fumarate | 3.0 |
| **Total core tablet** | **100** |
| Film coating | 5.66 |

### Example 3: Film-coated tablet

| **Components** | **Amount (%)** |
|---|---|
| Sitagliptin Malate | 33.23 |
| Microcrystalline cellulose | 40.77 |
| Dicalcium Phosphate Anhydrous | 20.0 |
| Croscarmellose sodium | 5.0 |
| Magnesium Stearate | 0.5 |
| Sodium stearyl fumarate | 0.5 |
| **Total core tablet** | **100** |
| Film coating | 5.6 |

A process for example 1 or 2 or 3;
a) Sieving sitagliptin or a pharmaceutically acceptable salt thereof, microcrystalline cellulose, dicalcium phosphate anhydrous and croscarmellose sodium through 630 µm and mixing,
b) Sieving magnesium stearate and sodium stearyl fumarate through 630 µm and mixing with the mixture at step (a),
c) Compressing the mixture into the tablet,
d) Coating the tablets with film coating agent.

## Claims

1. A film coated tablet comprises;
- Sitagliptin or a pharmaceutically acceptable salt thereof,
- At least two fillers,
- At least two lubricants,
Wherein, lubricants are magnesium stearate and sodium stearyl fumarate.

2. The film coated tablet according to claim 1, wherein the amount of sitagliptin or a pharmaceutically acceptable salt thereof is present between 20.0% and %45.0 by weight in the total core tablet.

3. The film coated tablet according to claim 1, wherein sitagliptin is in the form of phosphate monohydrate or malate.

4. The film coated tablet according to claim 1, wherein fillers are selected from the group comprising microcrystalline cellulose, dicalcium phosphate anhydrous, lactose, lactose monohydrate, spray-dried lactose monohydrate, anhydrous lactose, pregelatinized starch, mannitol, corn starch, maltodextrin, dicalcium phosphate, ammonium alginate, calcium carbonate, calcium phosphate, calcium phosphate dehydrate, neutral pellets, cellulose, magnesium oxide, maltodextrin, medium chain triglycerides, polydextrose, polymethacrylates, sodium alginate, starch, sugar sphericals, talc, polysorbate 80 or mixtures thereof.

5. The film coated tablet according to claim 1, wherein filler microcrystalline cellulose to dibasic calcium phosphate ratio is between 0.8:1 and 2.2:1.

6. The film coated tablet according to claim 1 or 4, wherein the amount of fillers is 50.0% to 75.0% by weight in the total core tablet.

7. The film coated tablet according to claim 1 or 4, wherein the fillers are microcrystalline cellulose and dicalcium phosphate anhydrous.

8. The film coated tablet according to claim 1, wherein Sitagliptin or a pharmaceutically acceptable salt thereof has a d (0.9) particle size more than 75 µm.

9. The film coated tablet according to claim 1, wherein sitagliptin or a pharmaceutically acceptable salt thereof has a d (0.9) particle size between 94 µm and 220 µm, preferably between 100 µm and 200 µm, preferably between 124 µm and 180 µm, preferably between 140 µm and 160 µm.

10. The film coated tablet according to claim 1 or 3, wherein Sitagliptin phosphate monohydrate has a d (0.9) particle size between 165 µm and 250 µm.

11. The film coated tablet according to claim 1 or 3, wherein Sitagliptin malate has a d (0.9) particle size between 75 µm and 150 µm.

12. The film coated tablet according to claim 1, wherein the film coated tablet further comprises at least one disintegrant.

13. The film coated tablet according to claim 1 or 11, wherein, the tablet comprises;
a) Sitagliptin Phosphate Monohydrate or Sitagliptin Malate
b) Microcrystalline Cellulose
c) Dicalcium Phosphate Anhydrous
d) Croscarmellose sodium
e) Magnesium stearate
f) Sodium stearyl fumarate

14. The film coated tablet according to claim 1 or 11, wherein the total composition comprises the following:
20.0-45.0% by weight of Sitagliptin Phosphate Monohydrate or Sitagliptin Malate
25.0-45.0% by weight of microcrystalline cellulose
15.0-35.0% by weight of dibasic calcium phosphate anhydrate
1.0-10.0% by weight of croscarmellose sodium
0.1-4.0% by weight of sodium stearyl fumarate
0.1-3.0% by weight of magnesium stearate by weight of the total core tablet.

15. The film coated tablet according to claim 1, wherein the film coated tablets are obtained by a process which is free of solvent.
